# EUROPEAN PATENT APPLICATION

(11) **EP 3 355 099 A1**
(43) Date of publication of application: **01.08.2018**
(21) Application number: 16848748.6
(22) Date of filing: 10.05.2016
(51) Int. Cl.: G02C 9/04, G02C 5/00, G02C 5/14, G02C 5/22, G02C 5/12, B29C 45/14

(54) **GOGGLES FOR PERSON NOT WEARING EYEGLASSES**

(30) Priority: 25.09.2015 KR 20150136595
(71) Applicant: Bang, Ki-Tae, Gangneung-si, Gangwon-do 25610 (KR)
(72) Inventor: Bang, Ki-Tae, Gangneung-si, Gangwon-do 25610 (KR)
(74) Representative: Zardi, Marco
(86) International application number: PCT/KR2016/004871
(87) International publication number: WO 2017/052022

(57) **Abstract**

The present invention relates to goggles and a method for manufacturing the same. The goggles include: a lens portion (3) configured to be integrated with a lens frame; side frames (7) configured to be integrated with both sides of the lens portion (3), and to come into tight contact with both sides of a face; folding members (8) configured to be disposed on the side frames (7), and to enable the side frames (7) to be selectively folded and unfolded; an elastic member (9) configured to be coupled to the rims of the lenses (3) and to come into contact with the face and prevent moisture; goggle supports (13) configured to be mounted on the lens portion (3), and to support the goggles; and nose pads (11) configured to be provided below the goggle supports (13), and to be supported on the tops of noses.

## Description

### Technical Field

The present invention relates to goggles for a person not wearing eyeglasses, and more specifically to goggles the wearing of which is made convenient and stable by improving the structure thereof in such a manner that lenses and temples are integrated with each other, folding members are mounted, and so forth.

### Background Art

Generally, sports goggles are classified into swimming goggles used in underwater environments, goggles used during the performance of sports, such as skiing, paragliding, etc., and goggles used for the purpose of safety at industrial sites.

Swimming goggles and other goggles are worn for the purpose of protecting the eyes of users. Each of the goggles includes a combination of various parts, including lenses, a frame integrated with the lenses, a pad configured to bring the goggles into tight contact with the surface of a face, and a band configured to fasten the goggles to a head.

Furthermore, the goggles used at industrial sites are used for the purpose of protecting eyes during work, such as welding or the like.

However, the conventional goggles have the following problems:
First, a problem arises in that although temples are caught on a user's ears, the conventional goggles do not sufficiently come into tight contact with the skin of a face and, thus, during wearing, the goggles may be easily removed from the face and may be moved during running.
Second, a problem arises in that the conventional goggles include a lens frame, lenses, and temples and, thus, an assembly process is complex and time-consuming.
Third, a problem arises in that, in the case where rubber is disposed along the edge of a lens frame, as in the conventional goggles, when the goggles are worn on a face, the rubber is compressed into the skin of the face and, thus, a user's view is blocked by moisture.

### Disclosure

### Technical Problem

The present invention has been conceived to overcome the above-described problems, and an object of the present invention is to provide goggles the manufacturing process of which is made simple by improving the structure thereof in such a manner that a lens frame, lenses and temples are integrated with each other and folding members are mounted.

Another object of the present invention is to provide goggles which are not humidified even when an elastic member is disposed along the edge of a lens frame.

### Technical Solution

In order to accomplish the above objects, an embodiment of the present invention provides goggles including:
a lens portion (3) configured to be integrated with a lens frame;
side frames (7) configured to be integrated with both sides of the lens portion (3), and to come into tight contact with both sides of a face;
folding members (8) configured to be disposed on the side frames (7), and to enable the side frames (7) to be selectively folded and unfolded;
an elastic member (9) configured to be coupled to the rims of the lenses (3) and to come into contact with the face and prevent moisture;
goggle supports (13) configured to be mounted on the lens portion (3), and to support the goggles; and
nose pads (11) configured to be provided below the goggle supports (13), and to be supported on the tops of noses.

### Advantageous Effects

As described above, the goggles and manufacturing method according to embodiments of the present invention have the following advantages:
First, an advantage arises in that the lenses and the side frames on both sides are formed through injection molding in an integrated manner and the folding members are mounted on the side frames, thereby simplifying a manufacturing process.
Second, an advantage arises in that the side frames on both sides elastically press both side surfaces of a face due to a structure in which the side frames are elastically coupled to the lenses during wearing even when the length of the side frames corresponding to glass temples is short, thereby enabling the goggles to be more stably worn on the face.
Third, an advantage arises in that goggles are not humidified due to the circulation of air attributable to the recess groove even when the elastic member is disposed along the edges of the lenses.

### Description of Drawings

FIG. 1 is a perspective view showing goggles according to an embodiment of the present invention;
FIG. 2 is a front view of FIG. 1;
FIG. 3 is a side view of FIG. 1;
FIG. 4 is a plan view of FIG. 1;
FIG. 5 is a front view of the elastic member shown in FIG. 1;
FIG. 6 is a rear view of the elastic member shown in FIG. 5;
FIG. 7 is a view showing a state in which the elastic member is in contact with a skin when viewed from line A-A of FIG. 2;
FIG. 8 is a perspective view showing goggles without folding members according another embodiment of the present invention;
FIG. 9 is a view showing a state in which a user has worn the goggles shown in FIG. 1; and

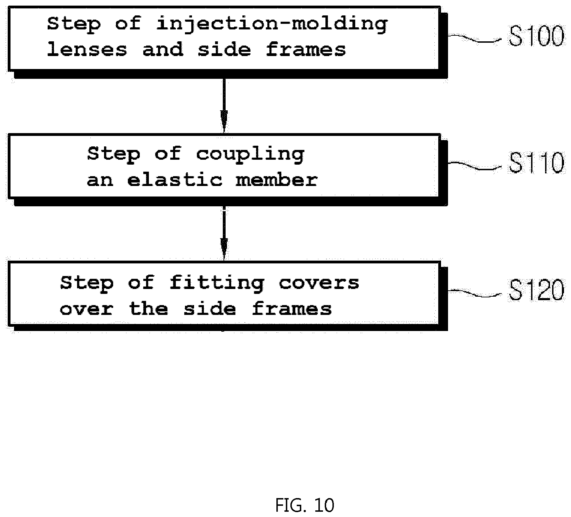

FIG. 10 is a flowchart showing a method for manufacturing goggles according to an embodiment of the present invention.

### Best Mode

Goggles according to embodiments of the present invention will be described in detail below with reference to the accompanying drawings.

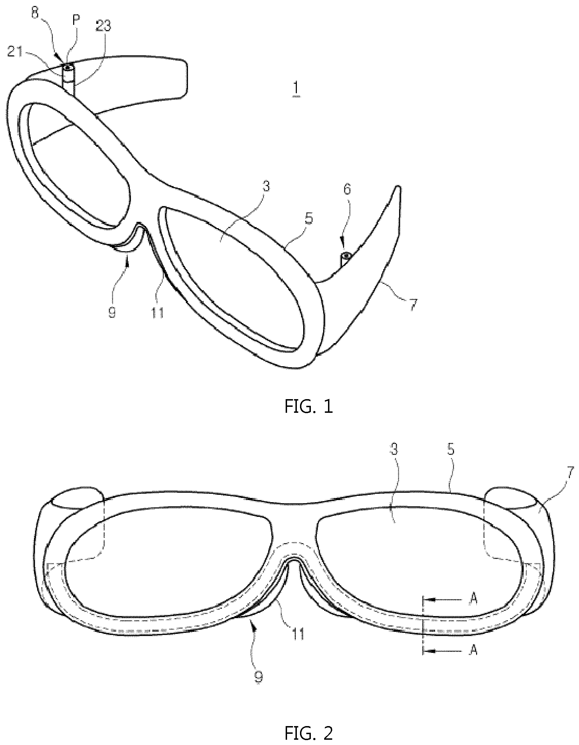

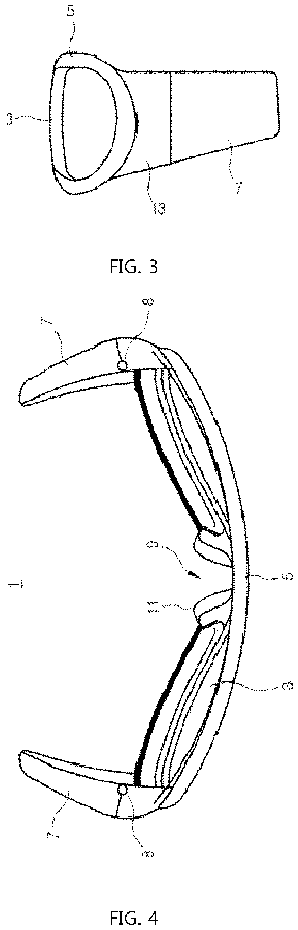

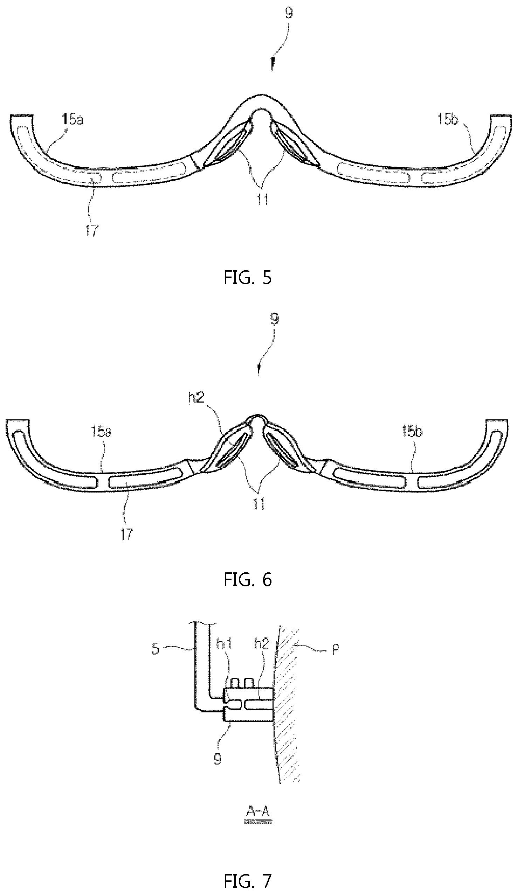

As shown in FIGS. 1 to 9, goggles 1 proposed by the present invention includes: lenses 3 configured to be integrated with a lens frame; side frames 7 configured to be integrated with both sides of the lenses 3, and to come into tight contact with both sides of a face; folding members 8 configured to be disposed on the side frames 7, and to enable the side frames 7 to be selectively folded and unfolded; and an elastic member 9 configured to be coupled to the rims of the lenses 3, and to come into contact with the face and prevent moisture.

The goggles 1 having the above structure have a structure in which a lens frame is omitted and only the lenses 3 and the side frames 7 are integrated with each other. In other words, when the goggles 1 are formed, the formation is performed using a method in which a shape corresponding to the lenses 3 and the side frames 7 is formed inside a mold, the raw material of the lenses is introduced into the mold, and the raw material is injected, as in a common injection molding process. As described above, the injection-molded goggles 1 have a structure in which a lens frame is omitted and only the lenses 3 and the side frames 7 are integrated with each other.

In this case, the raw material introduced into an injection molding machine is suitably synthetic resin, i.e., a transparent material, or the like in order to ensure the transparency of the lenses. Furthermore, this raw material preferably has elasticity because the side frames 7 on both sides need to come into elastic contact with both side surfaces of a face when the goggles 1 are worn on the face.

Furthermore, although the injection molding has been described above, the present invention is not limited thereto, but a molding method other than the injection molding may be applicable.

Furthermore, although the lenses 3 and rims 5 may be formed to have the same thickness, they may be formed to have different thicknesses through an appropriate change. For example, the lenses 3 may be formed to have a thickness of 2 mm, and the rims 5 may have a thickness of 3 mm. It will be apparent that the thickness of the lenses 3 may vary in various manners.

Accordingly, the goggles 1 formed as described above are configured such that the lenses 3 and the side frames 7 on both sides of the lenses 3 are integrated with each other, and have a "U" shape when viewed from above.

Furthermore, the elastic member 9 may be inserted over the rims 5 below the backs of the lenses 3 opposite to a face.

The side frames 7 are bent toward a backward direction (the direction of ears of the face) from both side ends of the lenses 3, and extend to a predetermined length. In this case, although the side frames 7 may have various lengths, they preferably have a length to the extent that the front ends thereof are located between the lenses 3 and the ears. In other words, the side frame 7 may be worn on the sides of the face only by means of elastic force without being worn on the ears.

Covers 7 may be coupled onto the outsides of the side frames 7. In other words, in order to improve a tactile sensation and increase a tight contact effect when the side frames 7 come into direct contact with the skin P of a face, the covers 7 made of a synthetic resin material, such as silicone or the like, may be fitted over the rear end portions of the side frames 7.

In this case, slits are formed inside the covers 7, and thus the covers 7 have a structure such that they may be inserted over the side frames 7. Furthermore, the surfaces of the covers 7 may be formed to be flat. Alternatively, friction force may be increased by forming protrusions and depressions on the surfaces of the covers 7, as desired. This may be appropriately selected.

The covers 7 are coupled onto the side frames 7 on both sides, respectively, and thus more stable tight contact is achieved when the goggles 1 are worn on a user, thereby preventing the goggles 1 from being shaken during movement or exercise.

Furthermore, the folding members 8 are mounted on the side frames 7, and thus the side frames 7 may be selectively folded and unfolded, as desired.

The folding members 8 include hinges having a common structure, etc. Each of the folding members 8 includes: a first folding part 21 configured to be mounted on one side of each of the side frames 7; a second folding part 23 configured to be mounted on the other side of the side frame 7; and a hinge pin P configured to couple the first and second folding parts 21 and 23 such that they are hinged together. Accordingly, the side frame 7 may be selectively folded and unfolded around the hinge pin P.

In this case, the first folding part 21 and both side ends of the lenses 3 without a lens frame may be injection-molded in an integrated form. Furthermore, the second folding part 16 and the side frame 7 may be injection-molded in an integrated form.

Accordingly, when the lenses 3 are coupled to the side frames 7, the side frames 7 may be coupled to the lenses 3 by coupling the first and second folding parts 21 and 23 to each other and then inserting the hinge pin P into the first and second folding parts 21 and 23.

Although the folding members have been described as being mounted on the side frames above, the present invention is not limited thereto, but the folding members may be omitted.

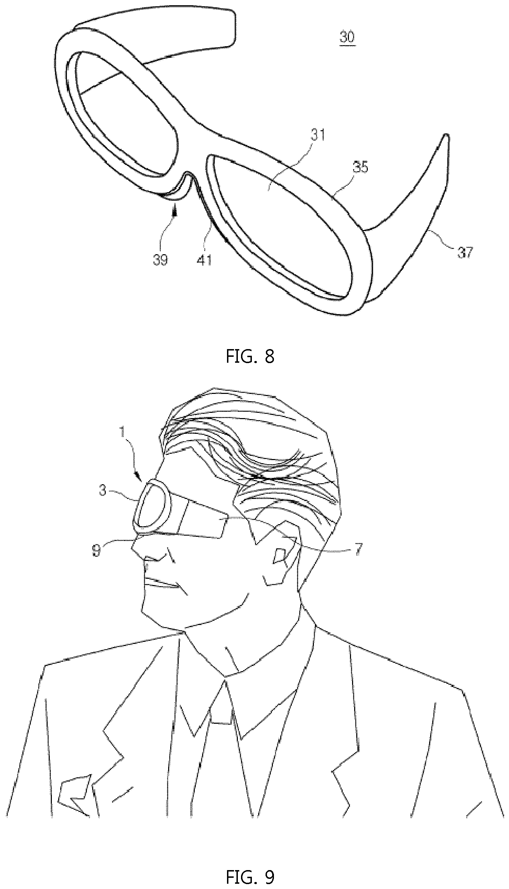

In other words, as shown in FIG. 8, there may be provided a structure in which the lenses 31 of goggles 30 and a lens frame 35 are formed in an integrated manner and both sides of the lenses 31 and side frames 37 are formed in an integrated manner. In this case, nose pads 41 and an elastic member 39 configured to come into contact with a face and prevent moisture may be mounted below the lenses 31.

Meanwhile, the elastic member 9 is mounted below the back surfaces of the lenses 3, and thus the lower portion of the goggles 1 may come into tight contact with the skin P of the face. This elastic member 9 may be made of various elastic materials. For example, the elastic member 9 may be made of a material, such as silicone. Furthermore, the elastic member 9 has a shape corresponding to the lower portion of the rim 5 of each of the lenses 3, and, thus, may be coupled to the back surfaces of the lenses 3.

In greater detail, the elastic member 9 includes: nose pads 11 configured to come into contact with and support noses; a first elastic portion 15a configured to extend from one side of the nose pads 11 and be coupled to the lower portion of the rim 5 of a first lens; and a second elastic portion 15b configured to extend from the other side of the nose pads 11 and be coupled to the lower portion of the rim 5 of a second lens.

A coupling groove h1 configured to receive the rims 5 of the lenses 3 is formed through the front surfaces (opposite to the lenses) of the first and second elastic portions 15b of the elastic member 9; and at least one recess groove h2 is formed through the back surfaces (opposite to the face) thereof.

Accordingly, the lenses 3 may be integrated with the elastic member 9 by inserting the rims 5 of the lenses 3 into the coupling groove h1 of the elastic member 9. Furthermore, when the goggles 1 are worn on a face, the elastic member 9 comes into contact with the skin P of a face. In this case, air may be circulated via the recess groove h2, and thus completely tight contact may be prevented.

Furthermore, the elastic member 9 may have various dimensions according to specifications. For example, the width of the elastic member 9 ranges from 8 to 20 mm, the depth of the coupling groove h1 into which the lens frame is inserted ranges from 2 to 10 mm, and the depth of the recess groove h2 which comes into contact with the skin P ranges from 5 to 20 mm. It will be apparent that changes to the above dimensions may be possible.

Furthermore, the nose pads 11 are disposed between the first elastic portion 15a and the second elastic portion 15b, and come into contact with the skins of the noses and support the weight of the goggles 1.

The nose pads 11 and the elastic member 9 are formed by injection molding in an integrated manner. The nose pads 11 include a pair of pads on both sides. A recess groove h2 is formed through the surfaces of the nose pads 11, and, thus, may prevent the nose pads 11 from coming into completely tight contact with the skin P due to the circulation of air.

In this case, the distance and angle between the pair of nose pads 11 may be appropriately changed. For example, the angle which is formed by the nose pads 11 with respect to a horizontal direction ranges from 60 to 85 degrees, the distance between the pair of nose pads 11 ranges from 5 to 20 mm, and the height ranges from 15 to 25 mm. It will be apparent that changes to the above ranges may be possible.

When the goggles 1 are worn on a face, as described above, the pair of side frames 7 come into elastic contact with both sides of the face, and thus the goggles may be easily worn.

Furthermore, supporting is performed by means of a triangular support method in which the side frames 7 on both sides elastically press the side surfaces of a face inward and the nose pads 11 support the lower portion of the face, and thus the goggles 1 can be more stably worn on the face, thereby preventing the goggles 1 from being separated during exercise.

A method for manufacturing the goggles 1 will be described in greater detail below with reference to the accompanying drawings.

As shown in the drawings, the method for manufacturing the goggles 1 proposed by the present invention includes: (a) step S100 of introducing a raw lens material into a mold having the shape of the lenses 3 and the side frames 7 and then injecting the raw lens material; (b) step S110 of coupling the elastic member 9 made of a flexible material to the lower portion of a lens part; and (c) step S120 of fitting the covers 7 over the side frames 7 of the goggles 1.

In this manufacturing method, at step S100, the lenses 3 and the side frames 7 are injection-molded. In other words, the mold having a shape identical to that of the lenses 3 and the side frames 7 is prepared.

Furthermore, the lenses 3 integrated with the side frames 7 may be formed by introducing a raw lens material into the mold and then pressing and heating the raw lens material, as in a common injection molding process.

In this case, the raw material introduced into an injection molding machine is preferably synthetic resin, i.e., a transparent material, or the like, in order to ensure the transparency of lenses. Furthermore, this raw material preferably has elasticity because the side frames 7 on both sides need to come into elastic contact with both side surfaces of a face when the goggles 1 are worn on the face.

After step (a) (step S100) has been completed as described above, step (b) (step S110) is performed. At this step, the elastic member 9 manufactured via a separate process is coupled to the lower portions of the lenses 3.

In other words, the coupling groove h1 is formed through the front surface of the elastic member 9. The elastic member 9 and the lenses 3 are fastened together in an integrated manner by inserting the lower portions of the rims 5 of the lenses 3 into the coupling groove h1.

In this case, the elastic member 9 may be coupled over a range from the side frame 7 at one end through the lower portions of the rims 5 of the lenses 3 to the side frame 7 at the other end.

After step (b) (step S110) has been completed as described above, step (c) (step S120) is performed. At this step, the covers 7 are fitted over the back end portions of the side frames 7. In other words, slits are formed inside the covers 7, and thus the covers 7 have a structure such that they may be inserted over the side frames 7. Accordingly, the covers 7 may be coupled onto the side frames 7 by inserting the covers 7 over the back end portions of the side frames 7.

When a user wears the goggles 1 manufactured via the above-described process, the goggles 1 are supported by means of a triangular support method in which the side frames 7 at both ends press both side surfaces of a face and also the nose pads support a portion below the lenses 3, and thus the goggles 1 may be more stably worn.

### Industrial Applicability

The present invention relates to goggles, and has industrial applicability in the field of goggles because the wearing of the goggles is made convenient and stable by improving the structure thereof in such a manner that the lenses and the side frames are integrated with each other, the folding members are mounted, and so forth.

## Claims

1. Goggles comprising:
lenses configured to be integrated with a lens frame;
side frames (7) configured to be integrated with both sides of the lenses (3), and to come into tight contact with both sides of a face;
folding members (8) configured to be disposed on the side frames (7), and to enable the side frames (7) to be selectively folded and unfolded;
an elastic member (9) configured to be coupled to rims of the lenses (3), and to come into contact with the face and prevent moisture;
goggle supports (13) configured to be mounted on the lenses (3), and to support the goggles; and
nose pads (11) configured to be provided below the goggle supports (13), and to be supported on tops of noses.

2. The goggles of claim 1, wherein:
the elastic member (9) comprises a pair of portions: a first elastic portion (15a) configured to be coupled to a lower portion of a rim (5) of a first lens; and a second elastic portion (15b) configured to be coupled to a lower portion of a rim (5) of a second lens; and
a coupling groove (h1) configured to receive the rims (5) of the lenses (3) is formed through front surfaces (opposite to the lenses) of the first and second elastic portions (15a and 15b) of the elastic member (9); and at least one recess groove (h2) is formed through back surfaces (opposite to a face) thereof.

3. The goggles of claim 1, wherein the goggle support (13) includes a base (19); a pair of vertical bars (20 and 24) configured to protrude above the base (19), to be spaced apart from each other by a predetermined distance, and to come into tight contact with a connection frame for the lenses (3) disposed therebetween; a horizontal bar (22) configured to protrude laterally from a top of one of the pair of vertical bars (20 and 24), and to come into contact with a top surface of the connection frame F for the lenses (3); and a catch protrusion (23) configured to protrude downward from a front end of the horizontal bar (22), and to fasten a frame of the worn goggles by catching it.

4. The goggles of claim 1, wherein the goggle support (13) comprises: an intermediate goggle support (13) configured to be mounted between the lenses (3); a first goggle support 40 configured to be mounted on a first lens portion (3); and a second goggle support (42) configured to be mounted on a second lens portion (3).

5. The goggles of claim 4, wherein each of the first and second goggle supports (40 and 42) comprises: a horizontal bar (22); a pair of vertical bars (20 and 24) configured to protrude below the horizontal bar (22), to be spaced apart from each other by a predetermined distance, and to come into tight contact with the lenses (3) disposed therebetween; and a catch protrusion (23) configured to protrude downward from a front end of the horizontal bar (22), and to fasten a frame of the worn goggles by catching it.

6. The goggles of claim 1, wherein the nose pads (11) comprise: a first pad (25) configured to protrude downward from one side of a lower portion of a base (19); and a second pad (27) disposed to be spaced apart from the first pad (25) by a predetermined distance.

7. The goggles of claim 1, wherein:
the lenses (3) additionally include a pair of lens fastening bars (30); and
at least two coupling grooves (34 and 36) are formed on bottoms of the lens fastening bars (30), so that one of the coupling grooves (34 and 36) is inserted over an upper portion of a rim of the lens portion (3) and the goggle lenses are inserted into the remaining coupling groove (36).

8. The goggles of claim 1, wherein each of the folding members (6) includes: a first folding part (21) configured to be mounted on one side of each of the side frames; a second folding part (23) configured to be mounted on a remaining side of the side frame; and a hinge pin (P) configured to couple the first and second folding parts (21 and 23) such that they are hinged together.
